Europäisches Patentamt

European Patent Office

Office européen des brevets

(11) Veröffentlichungsnummer: **0 255 843**
**A1**

(12) **EUROPÄISCHE PATENTANMELDUNG**

(21) Anmeldenummer: 86110823.1

(22) Anmeldetag: 05.08.86

(51) Int. Cl.⁴: **A61K 7/035** , **B65D 81/32**

(43) Veröffentlichungstag der Anmeldung:
17.02.88 Patentblatt 88/07

(84) Benannte Vertragsstaaten:
**AT BE CH DE FR GB IT LI LU NL SE**

(71) Anmelder: **Württembergische Parfümerie-Fabrik GmbH**
**Schillerstrasse 21-27**
**D-7332 Eislingen/Fils 16(DE)**

(72) Erfinder: **Scheller, Hans Ulrich, Dr.**
**Vogelgartenstrasse 45**
**D-7332 Eislingen/Fils(DE)**
Erfinder: **Scheller, Karl Alexander**
**Kelterstrasse 38**
**D-7332 Eislingen/Fils(DE)**

(74) Vertreter: **Werner, Hans-Karsten, Dr. et al**
**Deichmannhaus am Hauptbahnhof**
**D-5000 Köln 1(DE)**

(54) **Verfahren zur Herstellung von verschiedenfarbigen festen Puder-Zubereitungen.**

(57) Das Verfahren zur Herstellung von Behältnissen mit zwei oder mehreren voneinander getrennten verschiedenfarbigen Teilbereichen fester, gegossener Puder-Zubereitungen vewendet Behältnisse, in welcher Trennstege aus einer pastösen Masse eingespritzt wurden, welche eine ähnliche Zusammensetzung hat, wie die späteren festen gegossenen Puder-Zubereitungen. Die Stege verhindern ein Ineinanderlaufen verschiedenfarbiger Teilbereiche, verhalten sich jedoch dem Verbraucher gegenüber völlig neutral.

EP 0 255 843 A1

## Verfahren zur Herstellung von verschiedenfarbigen festen Puder-Zubereitungen

Gegenstand der vorliegenden Erfindung ist ein Verfahren zur Herstellung von Behältnissen mit zwei oder mehreren voneinander getrennten verschiedenfarbigen Teilbereichen fester, gegossener Puder-Zubereitungen. Insbesondere handelt es sich um voneinander getrennte verschiedenfarbige Teilbereiche fester, gegossener Puder-Zubereitungen gemäß der europäischen Patentanmeldung 84 108 763.8.

Behältnisse mit zwei oder mehreren voneinander getrennten verschiedenfarbigen Teilbereichen fester, gegossener Puder-Zubereitungen konnten bisher nur hergestellt werden, wenn mehrere entsprechend aneinanderpassende Behältnisse vorhanden waren oder in die Behältnisse vorher Trennstege eingebaut oder eingespritzt worden waren. Dies erfordert zumindest einen entsprechend hohen Aufwand für Spritzwerkzeuge bzw. die Montage von mehreren Aufnahmeformen (Pfannen) in die Behältnisse.

Die Erfindung hat sich die Aufgabe gestellt, ein Verfahren zu entwickeln, durch welches zwei oder mehrere voneinander getrennte verschiedenfarbige Teilbereiche fester Puder-Zubereitungen in Behältnisse gegossen werden können, ohne daß die Farben zusammenfließen und sich vermischen. Weiterhin bestand die Aufgabe, eine qualitative Veränderung der festen, gegossenen PuderZubereitungen zu vermeiden. Schließlich sollte das Verfahren möglichst einfach aber flexibel anwendbar sein, um nicht nur gerade sondern auch gekrümmte und geschwungene Trennlinien zwischen den verschiedenfarbigen Teilbereichen der Puder-Zubereitungen zu ermöglichen.

Diese Aufgabe konnte überraschend einfach dadurch gelöst werden, daß man spritzfähige pastöse Massen ent wickelt hat, die eine ähnliche Zusammensetzung aufweisen wie die fertigen festen gegossenen Puder-Zubereitungen und diese Massen dann als Trennstege in die Behältnisse einspritzt. Nach dem Einspritzen der pastösen Stege können in die einzelnen Teilbereiche der durch Stege unterteilten Behältnisse verschiedenfarbige Puder-Zubereitungen gegossen werden, woraufhin in üblicher Weise der Überschuß des Lösungsmittel verdampft und gewünschtenfalls die Oberfläche der so erhaltenen Produkte mit Drücken unter 10 bar bearbeitet wird.

Gegenstand der vorliegenden Erfindung ist somit ein Verfahren zur Herstellung von Behältnissen mit zwei oder mehreren voneinander getrennten, verschiedenfarbigen Teilbereichen fester, gegossener Puder-Zubereitungen, dadurch gekennzeichnet, daß in die Behältnisse

I. Trennstege eingespritzt werden aus einer pastösen Masse bestehend aus

    a) einem nicht hygroskopischen, rieselfähigen, organischen Feststoff mit einer Teilchengröße von 5 bis 50 $\mu$m Durchmesser als Füllstoff,

    b) einem oder mehreren fettartigen und wachsartigen Bestandteilen,

    c) einem farbneutralen Pigment oder Perlpigment,

    d) einem oder mehreren verdampfbaren untoxischen, hydrophoben Lösungsmitteln,

    e) gegebenenfalls weiteren üblichen Zusätzen für gießbare feste Puder-Zubereitungen, worauf

II. in die einzelnen Teilbereiche der durch Stege unterteilten Behältnisse verschiedenfarbige Puder-Zubereitungen gegossen werden, worauf

III. der Überschuß des Lösungsmittels verdampft und gewünschtenfalls die Oberfläche der so erhaltenen Produkte mit Drücken unter 10 bar bearbeitet wird.

Da die Zusammensetzung der Zusammensetzung inzwischen handelsüblicher fester, gegossener Pulver-Zubereitungen gemäß europäischer Patentanmeldung 84 108 763.8 entsprechen soll, wird vorzugsweise als Füllstoff Nylon-12-Pulver verwendet, bestehend aus überwiegend kugelförmigen, ellipsoiden Körpern. Das Schüttgewicht beträgt meist 0,20 bis 0,35. Die Dichte liegt meist zwischen 1,02 bis 1,03. Eingesetzt wird dieses Nylon-12-Pulver in Mengen zwischen 7,0 bis 15 Gew.-%, vorzugsweise ca. 8 Gew.-%.

Als fettartige und wachsartige Bestandteile werden vorzugsweise Oleyl Erucate, Stearinsäure-mono-ethanolamid und Glyceryl Oleate verwendet. Die Mengen liegen vorzugsweise zwischen 3,0 bis 5, insbesondere ca. 4 Gew.-% Oleyl Erucat, 0,8 bis 2,0 Gew.-%, insbesondere ca. 1 Gew.-% Stearinsäure-mono-ethanolamid, 0,4 bis 2,0 Gew.-%, insbesondere ca. 0,5 Gew.-% Glycerylstearat und/oder Oleat.

Die Mengen an farbneutralem Pigment bzw. Perlpigment liegen vorzugsweise zwischen 40 und 50 Gew.-%, insbesondere bei ca. 43 Gew.-%.

Als verdampfbares untoxisches hydrophobes Lösungsmittel wird vorzugsweise Octamethylcyclotetrasiloxan eingesetzt und zwar in Mengen von 35,0 bis 45,0 Gew.-%.

Als weitere übliche Zusätze für gießbare feste PuderZubereitungen werden auch erfindungsgemäß ca. 0,1 Gew.-% Methylparaban, ca. 0,1 Gew.-% Propylparaban, ca 0,1 Gew.-% Methylchloroisothiazolinone und Methylisothiazolinone sowie ca. 0,05 Gew.-% Butylhydroxytolulol eingesetzt.

Im Vergleich zu den üblichen Rezepturen für die zu gießenden festen Puder-Zubereitungen weisen somit die Rezepturen für die spritzfähigen pastösen Massen weniger fettartige und wachsartige Bestandteile und weniger Nylon-Pulver auf, jedoch einen höheren Anteil Pigment bzw. Perlpigment. Dadurch entstehen nicht fließbare, jedoch strangbildende pastöse Produkte, aus denen aus einer Spritzdüse Stege in die Behältnisse gespritzt werden können. Diese Stege können gerade, aber auch gebogen oder gekrümmt sein. Diese pastösen Stege schmelzen auch bei späteren Arbeitstemperaturen bis zu 80°C nicht wieder auf. Sie sind jedoch in der Lage, sich mit den flüssigen, gegossenen Puder-Zubereitungen nach dem Trocknungsvorgang zu einem einheitlichen, festen Puder zu verbinden. Sie bilden meist sogar eine optisch nicht oder kaum noch sichtbare Trennschicht, die sich bei der späteren Verwendung der Puder-Zubereitung in den Behältnissen völlig neutral verhalten. Da sie keine Farbpigmente, sondern nur farbneutrale Pigmente bzw. Perlpigmente enthalten, wird keine Vermischung der Farben beobachtet.

Nach dem erfindungsgemäßen Verfahren ist es somit möglich, in einem größeren Behältnis zwei oder mehrere voneinander getrennte verschiedenfarbige Teilbereiche herzustellen und dabei ansprechende Formen und Ornamente herzustellen. Das Verfahren ist dabei einfacher und preiswerter, als wenn Behältnisse mit Trennstegen hergestellt werden müßten, zumal für jede einzelne Formgebung neue Spritzwerkzeuge oder Tiefziehformen zur Verfügung gestellt werden müßten.

In dem nachfolgenden Beispiel ist eine bevorzugte Rezeptur für die spritzfähige pastöse Masse angegeben, welche insbesondere für die bereits auf dem Markt befindlichen Handelsprodukte geeignet ist. Prinzipiell ist es aber möglich, gemäß europäischer Patentanmeldung 84 108 763.8 verschiedenartige feste, gegossene PuderZubereitungen herzustellen, so daß auch daran angepaßte andere spritzfähige pastöse Massen zusammengestellt und verwendet werden können.

Beispiel

Eine spritzfähige pastöse Masse zum Gießen von Stegen in Behältnisse hat folgende Zusammensetzung:

| | |
|---|---|
| Oleyl Erucate | 4,0 % |
| Stearinsäure-mono-ethanolamid | 1,0 % |
| Glycerylstearat /oder Oleate | 0,5 % |
| Nylon-12-Pulver | 8,0 % |
| farbneutrales Pigment/ Perlpigment | 43,15% |
| Octamethylcyclotetrasiloxan | 43,0 % |
| Methylhydroxytoluol | 0,05% |
| Methlychloroisothiazolinone + Methylisothiazolinone | 0,10% |
| Methylparaban | 0,10% |
| Propylparaban | 0,10% |
| | 100,00% |

Dieses Gemisch kann bei Raumtemperatur oder Temperaturen bis zu 40° aus einer Düse strangförmig in Behältnisse eingebracht werden, und zwar mit einem Strangdurchmesser der etwa der Schichtdicke des späteren Puders entspricht. Unmittelbar danach können sie in an sich bekannter Weise mit verschiedenfarbigen Puder-Zubereitungen ausgegossen werden. Die Behältnisse werden in einem Trockenschrank bei 60°C schichtweise eingelagert und hierin etwa 5 bis 10 Stunden belassen, wobei der größte Anteil des Octamethylcyclotetrasiloxans verdampft. Gewünschtenfalls wird die Oberfläche der so erhaltenen Produkte mit Drücken unter 10 bar bearbeitet.

## Ansprüche

1. Verfahren zur Herstellung von Behältnissen mit zwei oder mehreren voneinander getrennten, verschiedenfarbigen Teilbereichen fester, gegossener Puder-Zubereitungen, dadurch gekennzeichnet, daß in die Behältnisse

I. Trennstege eingespritzt werden aus einer pastösen Masse bestehend aus

a) einem nicht hygroskopischen, rieselfähigen, organischen Feststoff mit einer Teilchengröße von 5 bis 50 μm Durchmesser als Füllstoff,

b) einem oder mehreren fettartigen und wachsartigen Bestandteilen,

c) einem farbneutralen Pigment oder Perlpigment,

d) einem oder mehreren verdampfbaren untoxischen, hydrophoben Lösungsmitteln,

e) gegebenenfalls weiteren üblichen Zusätzen für gießbare feste Puder-Zubereitungen, worauf

II. in die einzelnen Teilbereiche der durch Stege unterteilten Behältnisse verschiedenfarbige Puder-Zubereitungen gegossen werden, worauf

III. der Überschuß des Lösungsmittels verdampft und gewünschtenfalls die Oberfläche der so erhaltenen Produk te mit Drücken unter 10 bar bearbeitet wird.

2. Verfahren gemäß Anspruch 1, dadurch gekennzeichnet, daß als a) Nylon-12-Pulver verwendet wird, bestehend aus überwiegend kugelförmigen und ellipsoiden Körpern.

3. Verfahren gemäß Anspruch 1 oder 2, dadurch gekennzeichnet, daß als b) Gemische verwendet werden aus Oleyl Erucate, Stearinsäure-mono-ethanolamid und Glyceryl Oleate.

4. Verfahren gemäß einem der Ansprüche 1 bis 3, dadurch gekennzeichnet, daß als d) verwendet wird Octamethylcyclotetrasiloxan.

5. Verfahren gemäß einem der Ansprüche 1 bis 4, dadurch gekennzeichnet, daß als e) verwendet wird Methylparaban, Propylparaban, Butylhydroxytoluol und Methylchloroisothiazolinone/Methylisothiazolinone.

6. Verfahren gemäß einem der Ansprüche 1 bis 5, dadurch gekennzeichnet, daß als pastöse Masse verwendet wird ein Gemisch aus

a) 7,0 bis 15,0 Gew.-% Nylon-12-Pulver,

b) 3,0 bis 5,0 Gew.-% Oleyl Erucate,

0,8 bis 2,0 Gew.-% Stearinsäure-mono-ethanolamid,

0,4 bis 2,0 Gew.-% Glycerylstearate und/oder Oleate,

c) 40 bis 50 Gew.-% farbneutrales Pigment und/oder Perlpigment,

d) 35 bis 45 Gew.-% Octamethylcyclotetrasiloxan,

e) ca. 0,1 Gew.-% Methylparaban,

ca. 0,1 Gew.-% Propylparaban,

ca. 0,1 Gew.-% Methylchloroisothiazolinone und Methylisothiazolinone,

ca. 0,05 Gew.-% Butylhydroxytoluol.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | DE-A-2 349 051 (BEIERSDORF) <br> * Patentanspruch * <br><br> --- | 1 | A 61 K 7/035 <br> B 65 D 81/32 |
| D,A | EP-A-0 135 060 (WÜRTTEMBERGISCHE PARFÜMERIE-FABRIK) <br> * Patentansprüche; Beispiele * <br><br> ----- | 1-6 | |

RECHERCHIERTE
SACHGEBIETE (Int. Cl.4)

A 61 K 7/00
A 61 K 9/00
B 65 D 77/00
B 65 D 81/00
B 65 B 29/00

Der vorliegende Recherchenbericht wurde für alle Patentansprüche erstellt.

| Recherchenort <br> DEN HAAG | Abschlußdatum der Recherche <br> 27-04-1987 | Prüfer <br> WILLEKENS G.E.J. |
|---|---|---|